# FASCICULE DE BREVET EUROPEEN

(11) **EP 1 817 363 B1**
(45) Date de publication et mention de la délivrance du brevet: **06.01.2010**
(21) Numéro de dépôt: 05810748.3
(22) Date de dépôt: 03.10.2005
(51) Int. Cl.: C08G 77/04, C08G 77/26, C08G 77/388, G01N 27/00

(54) **CAPTEURS CHIMIQUES COMPRENANT DES POLYSILOXANES ANILINES COMME MATERIAUX SENSIBLES ET LEUR UTILISATION POUR LA DETECTION OU LE DOSAGE DE COMPOSES NITRES**
CHEMISCHE SENSOREN, DIE ANILINPOLYSILOXANE ALS EMPFINDLICHE SUBSTANZEN ENTHALTEN, UND IHRE VERWENDUNG ZUM NACHWEIS UND ZUR QUANTITATIVEN ANALYSE VON NITROVERBINDUNGEN
CHEMICAL SENSORS COMPRISING ANILINE POLYSILOXANES AS SENSITIVE MATERIALS AND USE THEREOF FOR DETECTING OF ASSAYING NITRO COMPOUNDS

(30) Priorité: 07.10.2004 FR 0452302
(43) Date de publication de la demande: 15.08.2007
(73) Titulaire: Commissariat à l'Energie Atomique, 75015 Paris (FR)
(72) Inventeur: POULLAIN, Didier, F-37550 ST AVERTIN (FR); PASQUINET, Eric, F-37550 ST AVERTIN (FR); HAIRAULT, Lionel, F-37150 BLERE (FR)
(74) Mandataire: Poulin, Gérard
(86) Numéro de dépôt international: PCT/FR2005/050805
(87) Numéro de publication internationale: WO 2006/037921

(56) Documents cités:
- US-A- 4 634 756
- MCGILL R A ET AL: "The design of functionalized silicone polymers for chemical sensor detection of nitroaromatic compounds" SENSORS AND ACTUATORS B, ELSEVIER SEQUOIA S.A., LAUSANNE, CH, vol. 65, no. 1-3, 30 juin 2000 (2000-06-30), pages 5-9, XP004208582 ISSN: 0925-4005 cité dans la demande
- ZHOU R ET AL: "Silicon-containing monomers, oligomers and polymers as sensitive coatings for the detection of organic solvent vapors" SENSORS AND ACTUATORS B, ELSEVIER SEQUOIA S.A., LAUSANNE, CH, vol. 26, no. 1-3, 1995, pages 121-125, XP004318438 ISSN: 0925-4005

## Description

### DOMAINE TECHNIQUE

La présente invention se rapporte à des capteurs chimiques comprenant des polysiloxanes anilinés en tant que matériaux sensibles ainsi qu'à l'utilisation de ces capteurs pour détecter ou doser des composés nitrés, en particulier des composés nitroaromatiques tels que le nitrobenzène (NB), le dinitrobenzène (DNB), le trinitrobenzène (TNB), le nitrotoluène (NT), le dinitrotoluène (DNT), le 2,4,6-trinitrotoluène (TNT) et analogues.

De tels capteurs sont utiles pour la détection d'explosifs, que ce soit en vue d'assurer la sécurité de lieux publics comme les aéroports, de contrôler la licéité de marchandises en circulation sur un territoire, de lutter contre le terrorisme, de procéder à des opérations de désarmement, de localiser des mines antipersonnel ou encore de dépolluer des sites industriels ou militaires.

Ils sont également utiles pour la protection de l'environnement, en particulier pour le contrôle et la surveillance de la pollution atmosphérique et de la qualité d'ambiances plus ou moins confinées, ainsi que pour la surveillance à des fins sécuritaires, de sites industriels fabriquant, stockant et/ou manipulant des composés nitrés.

### ÉTAT DE LA TECHNIQUE ANTÉRIEURE

La détection d'explosifs est un problème d'intérêt crucial, notamment en matière de sécurité civile.

A l'heure actuelle, plusieurs méthodes sont utilisées pour détecter des vapeurs de composés nitrés entrant dans la constitution des explosifs, comme l'emploi de chiens *"renifleurs"* dressés et entraînés à cet effet, l'analyse en laboratoire, par exemple par chromatographie couplée à un spectromètre de masse ou à un détecteur à capture d'électrons, d'échantillons prélevés sur site, ou encore la détection infrarouge.

Ces méthodes font, d'une manière générale, preuve d'une grande sensibilité, ce qui est primordial en matière de détection d'explosifs compte tenu de la très faible concentration en vapeurs de composés nitrés qui règne au voisinage d'un explosif. Elles ne donnent toutefois pas totalement satisfaction.

Ainsi, l'utilisation de chiens *"renifleurs"* présente l'inconvénient de nécessiter une longue formation des chiens et de leurs maîtres et d'être inadaptée à des opérations prolongées en raison de ce que la durée d'attention des chiens est limitée.

Quant aux autres méthodes, l'encombrement des appareillages qu'elles utilisent, leur consommation d'énergie et leurs coûts de mise en oeuvre s'opposent au développement de systèmes de détection aisément transportables et autonomes et, partant, aptes à être utilisés sur tout type de sites.

Depuis quelques années, le développement de capteurs capables de détecter en temps réel des espèces chimiques gazeuses est en plein essor. Le fonctionnement de ces capteurs est basé sur l'utilisation d'un film d'un matériau sensible, c'est-à-dire d'un matériau dont au moins une propriété physique P (masse, température, conductivité électrique, absorbance, fluorescence, ...) est modifiée au contact des molécules gazeuses recherchées, qui revêt un système apte à mesurer en temps réel toute variation de cette propriété physique et de mettre ainsi en évidence la présence des molécules gazeuses recherchées.

Les avantages des capteurs chimiques par rapport aux méthodes précitées sont multiples : instantanéité des résultats, possibilité de miniaturisation et, donc, portabilité, maniabilité et autonomie importante, faibles coûts de fabrication et d'exploitation, etc.

Toutefois, il est évident que leurs performances sont extrêmement variables selon la nature du matériau sensible utilisé.

Pour la détection de composés nitrés gazeux, et plus particulièrement de composés nitroaromatiques, de nombreux matériaux sensibles ont déjà été proposés parmi lesquels on peut citer le silicium poreux, le charbon végétal, le polyéthylène glycol, des amines, des cyclodextrines, des cavitands et des composés fluorescents.

Il a également été proposé par Briglin et al. dans Proceedings of SPIE, vol. 4394, 2001, 912-921, **[1]** d'utiliser, pour détecter le dinitrotoluène, des composites constitués de noir de carbone et de poly[bis(cyanoallyl)siloxane] et de noir de carbone et de polyméthyloctadécylsiloxane dans un système multicapteur basé sur la mesure d'une variation de la conductivité électrique de ces composites.

Par ailleurs, McGill et al. ont décrit dans Sens. Actuators B65, 5-9, 2000, **[2],** et dans la demande internationale PCT WO-A-02/08314 **[3],** la détection de quelques composés nitroaromatiques (nitrobenzène, dinitrotoluène, trinitrotoluène) par un capteur à ondes de surface, comprenant des polysiloxanes fonctionnalisés comme matériaux sensibles. Ces polysiloxanes sont fonctionnalisés par un groupe phényle substitué par un ou plusieurs groupes hexafluoroisopropanol (HFIP).

Selon McGill et *al.,* la présence de ce ou ces groupes HFIP est directement responsable de la sensibilité du capteur aux composés nitroaromatiques en raison de ce qu'elle permet la formation de liaisons hydrogènes entre la fonction hydroxyle présente dans ces groupes et le groupe nitro des composés nitroaromatiques.

Or, dans le cadre de leurs travaux sur le développement de capteurs destinés plus spécialement à détecter des explosifs, les Inventeurs ont constaté que des capteurs utilisant comme matériaux sensibles, des polymères à base de siloxanes fonctionnalisés par un groupe phényle substitué par une ou plusieurs fonctions amines (primaires, secondaires et/ou tertiaires) détectent les composés nitrés, et en particulier les composés nitroaromatiques, avec une sensibilité nettement plus élevée que des capteurs utilisant les polysiloxanes préconisés par McGill et *al.*, y compris dans le cas où ce groupe phényle ne comporte pas de groupe HFIP pendant.

Et c'est cette constatation qui est à la base de l'invention.

### EXPOSÉ DE L'INVENTION

L'invention a pour objet un capteur chimique qui comprend, en tant que matériau sensible, au moins un polymère comprenant un motif répétitif siloxane de formule (I) ci-après : 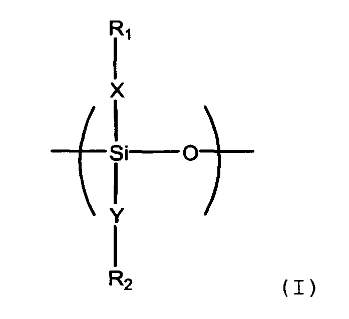 dans laquelle :
- X et Y représentent, indépendamment l'un de l'autre, une liaison simple ou un groupe hydrocarboné linéaire, ramifié ou cyclique, saturé ou insaturé, comprenant de 1 à 30 atomes de carbone et éventuellement un ou plusieurs hétéroatomes et/ou une ou plusieurs fonctions chimiques comportant au moins un hétéroatome et/ou un ou plusieurs groupes aromatiques ou hétéroaromatiques ;
- R₁ représente un groupe aniline répondant à la formule (II) ci-après : 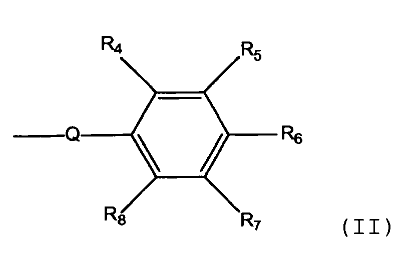 dans laquelle :
   • Q représente une liaison simple, un groupe -CH₂-, ou bien un groupe -NR₃- dans lequel R₃ représente un atome d'hydrogène ou un groupe hydrocarboné linéaire, saturé ou insaturé, et comprenant de 1 à 10 atomes de carbone ;
   • R₄ à R₈ représentent, indépendamment les uns des autres, un atome d'hydrogène, une fonction chimique comportant au moins un hétéroatome, ou bien un groupe hydrocarboné linéaire, ramifié ou cyclique, saturé ou insaturé, comprenant de 1 à 30 atomes de carbone et éventuellement un ou plusieurs hétéroatomes et/ou une ou plusieurs fonctions chimiques comportant au moins un hétéroatome et/ou un ou plusieurs groupes aromatiques ou hétéro-aromatiques ;
   et dans laquelle, lorsque Q est une liaison simple ou un groupe -CH₂-, alors au moins l'un des radicaux R₄ à R₈ représente un groupe aminé -NR₉R₁₀ dans lequel R₉ et R₁₀ représentent, indépendamment l'un de l'autre, un atome d'hydrogène, une fonction chimique comportant au moins un hétéroatome, ou bien un groupe hydrocarboné linéaire, ramifié ou cyclique, saturé ou insaturé, comprenant de 1 à 30 atomes de carbone et éventuellement un ou plusieurs hétéroatomes et/ou une ou plusieurs fonctions chimiques comportant au moins un hétéroatome et/ou un ou plusieurs groupes aromatiques ou hétéro-aromatiques ;
- R₂ représente un atome d'hydrogène ou un groupe aniline de formule (II) telle que définie ci-dessus.

Dans la formule (I) ci-avant, lorsque X et/ou Y représentent une liaison simple, alors R₁ et/ou R₂ sont liés directement à l'atome de silicium par une liaison covalente.

De manière analogue, lorsque R₁ représente un groupe de formule (II) dans laquelle Q représente une liaison simple, alors le noyau phényle de ce groupe est directement lié à X ou à l'atome de silicium si X représente lui-même une liaison simple, tandis que, lorsque R₂ représente un groupe de formule (II) dans laquelle Q représente une liaison simple, alors le noyau phényle de ce groupe est directement lié à Y ou à l'atome de silicium si Y représente lui-même une liaison simple.

Par ailleurs, lorsque X et/ou Y représentent un groupe hydrocarboné comprenant au moins deux atomes de carbone et que ce groupe comporte un ou plusieurs hétéroatomes et/ou une ou plusieurs fonctions chimiques et/ou un ou plusieurs groupes aromatiques ou hétéroaromatiques, alors cet(ces) hétéroatome(s), cette (ces) fonction (s) chimique (s) et ce (s) groupe (s) aromatique(s) ou hétéroaromatique(s) peuvent former pont à l'intérieur de ce groupe ou être portés latéralement par lui, tandis que, lorsque R¹, R², R³, R⁴, R⁵, R⁶, R⁷, R⁸, R⁹, R¹⁰, R¹¹ et/ou R¹² représentent un groupe hydrocarboné comprenant au moins deux atomes de carbone et que ce groupe comporte un ou plusieurs hétéroatomes et/ou une ou plusieurs fonctions chimiques et/ou un ou plusieurs groupes aromatiques ou hétéroaromatiques, alors cet(ces) hétéroatome(s), cette (ces) fonction(s) chimique(s) et ce(s) groupe(s) aromatique(s) ou hétéroaromatique(s) peuvent former pont à l'intérieur de ce groupe, être portés latéralement par lui ou encore se situer à son extrémité.

Conformément à l'invention, le ou les groupes hydrocarbonés entrant dans les formules (I) et (II) ci-avant, lorsqu'ils sont cycliques et insaturés, peuvent aussi bien être des groupes aromatiques (ou hétéroaromatiques s'ils comportent un ou plusieurs hétéroatomes dans leur cycle ou dans l'un des cycles qui les constituent), que des groupes non aromatiques (cycloalcéniques ou cycloalcyniques).

A titre d'exemples de groupes aromatiques susceptibles d'être utilisés dans l'invention, on peut citer les groupes cyclopentadiényle, phényle, benzyle, biphényle, phénylacétylényle, pyrène ou anthracène, tandis qu'à titre d'exemples de groupes hétéro-aromatiques, on peut citer les groupes furanyle, pyrrolyle, thiophényle, oxazolyle, pyrazolyle, thiazolyle, imidazolyle, triazolyle, pyridinyle, pyranyle, quinoléinyle, pyrazinyle et pyrimidinyle.

Le ou les hétéroatomes peuvent être tout atome autre que de carbone ou d'hydrogène comme, par exemple, un atome d'oxygène, de soufre, d'azote, de fluor, de chlore, de phosphore, de bore ou encore de silicium, les atomes d'oxygène, de soufre et d'azote étant préférés.

La ou les fonctions chimiques comportant au moins un hétéroatome peuvent notamment être choisies parmi les fonctions -COOH, -COOR₁₁, -CHO, -CO-, -OH, -OR₁₁, -SH, -SR₁₁, -SO₂R₁₁, -NH₂, -NHR₁₁, -NR₁₁R₁₂, -CONH₂, -CONHR₁₁, -CONR₁₁R₁₂, -C(Hal)₃, -OC(Hal)₃, -C(O)Hal, -CN, -COOCHO et -COOCOR₁₁, dans lesquelles :
- R₁₁ représente un groupe hydrocarboné, linéaire, ramifié ou cyclique, saturé ou insaturé, comprenant de 1 à 30 atomes de carbone, ou une liaison covalente dans le cas où ladite fonction chimique forme pont dans un groupe hydrocarboné en C₂ à C₃₀ ;
- R₁₂ représente un groupe hydrocarboné, linéaire, ramifié ou cyclique, saturé ou insaturé, comprenant de 1 à 30 atomes de carbone, ce groupe pouvant être identique ou différent du groupe hydrocarboné représenté par R₁₁ ; tandis que
- Hal représente un atome d'halogène, par exemple un atome de fluor, de chlore ou de brome.

Selon une première disposition préférée de l'invention, le motif répétitif siloxane répond à la formule (I) dans laquelle R₁ représente un groupe aniline de formule (IIa), (IIb) ou (IIc) ci-après :

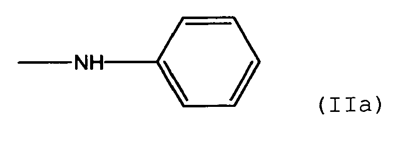

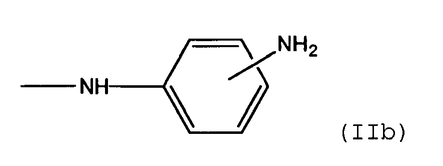

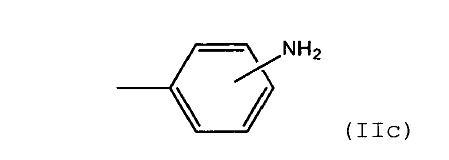

Dans les formules (IIb) et (IIc), le groupe amine primaire du groupe aniline peut se situer en position ortho, méta ou para par rapport à l'élément auquel est rattaché le noyau phényle, c'est-à-dire, dans le cas de la formule (IIb), par rapport au groupe amine secondaire de ce groupe aniline et, dans le cas de la formule (IIc), par rapport à X ou à l'atome de silicium si X représente une liaison simple.

Selon une autre disposition préférée de l'invention, le motif répétitif siloxane répond à la formule (I) dans laquelle X représente un groupe alkylène comprenant de 1 à 10 atomes de carbone et, de préférence, un groupe propylène, Y représente un groupe alkylène comprenant de 1 à 3 atomes de carbone et, de préférence, un groupe méthylène, tandis que R₂ représente un atome d'hydrogène.

Ainsi, parmi les motifs répétitifs siloxane de formule (I), on préfère notamment ceux dans lesquels X représente un groupe propylène, R₁ représente un groupe aniline de formule (IIa), (IIb) ou (IIc), Y représente un groupe méthylène, tandis que R₂ représente un atome d'hydrogène, et parmi eux, ceux répondant aux formules (Ia), (Ib) ou (Ic) ci-après :

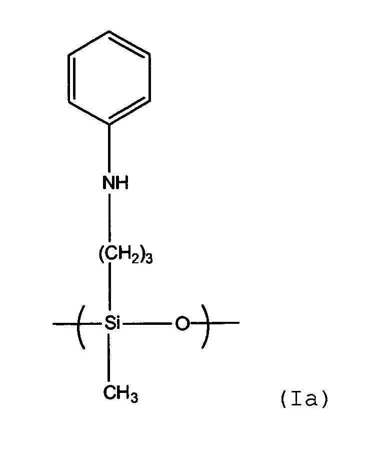

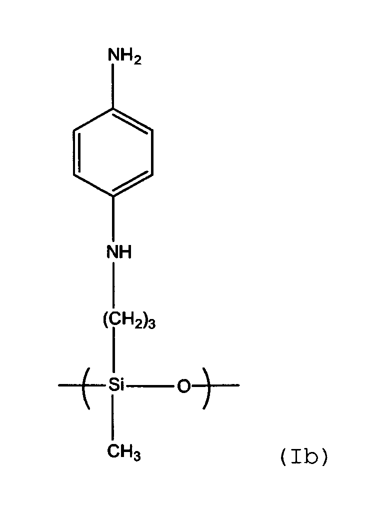

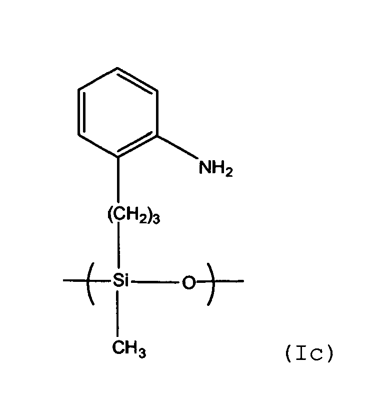

Conformément à l'invention, le polymère peut être un homopolymère, auquel cas il n'est constitué que d'un seul et même motif répétitif siloxane de formule (I).

Un tel homopolymère est, par exemple, un homopolymère formé par le motif répétitif de formule (Ia).

En variante, le polymère peut également être un copolymère, auquel cas il peut aussi bien être constitué de différents motifs répétitifs siloxane répondant tous à la formule (I) que comprendre un ou plusieurs motifs répétitifs siloxane de formule (I) et un ou plusieurs motifs répétitifs autres, siloxane ou non.

Selon encore une autre disposition préférée de l'invention, le polymère est un copolymère qui comprend deux motifs répétitifs siloxane différents, un premier motif siloxane répondant à la formule (I) ci-avant dans laquelle X, Y, R₁ et R₂ ont la même signification que précédemment, et un deuxième motif siloxane répondant à la formule (III) ci-après : 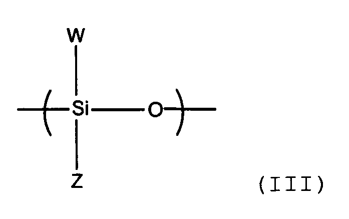 dans laquelle W et Z, qui peuvent être identiques ou différents, représentent un atome d'hydrogène, un groupe hydrocarboné linéaire, ramifié ou cyclique, saturé ou insaturé, comportant de 1 à 30 atomes de carbone.

De préférence, le deuxième motif siloxane est un dihydrogénosiloxane (W = Z = H), un méthylhydrogénosiloxane (l'un de W et de Z = H, tandis que l'autre = CH₃) ou un diméthylsiloxane (W = Z = CH₃), ce dernier motif étant préféré.

Des exemples de tels copolymères sont notamment des copolymères comprenant :
- un premier motif siloxane de formule (Ia) et un deuxième motif diméthylsiloxane ; ou
- un premier motif siloxane de formule (Ib) et un deuxième motif diméthylsiloxane ; ou encore
- un premier motif siloxane de formule (Ic) et un deuxième motif diméthylsiloxane.

Conformément à l'invention, ces copolymères peuvent être aléatoires, alternés ou séquencés, mais ils sont préférentiellement aléatoires.

Par ailleurs, on préfère les copolymères dans lesquels le premier motif siloxane représente, en nombre, environ 47% des motifs répétitifs formant ces copolymères, tandis que le deuxième motif siloxane représente, en nombre, environ 53% des motifs répétitifs formant lesdits copolymères.

Qu'il s'agisse d'un homopolymère ou d'un copolymère, la masse moléculaire du polymère en masse est généralement supérieure ou égale à 200 g/mole, et est, de préférence, de 200 à 100 000 g/mole et, mieux encore, de 1 000 à 10 000 g/mole.

Le polymère peut aussi comprendre un motif répétitif issu d'un monomère du type éthylène, propylène, oxyde d'éthylène, styrène, carbazole de vinyle ou encore acétate de vinyle, apte à lui conférer des propriétés mécaniques plus élevées dans le cas notamment où l'on souhaite l'utiliser sous la forme d'un film mince.

Selon encore une autre disposition préférée de l'invention, le polymère se présente sous la forme d'un film mince qui recouvre l'une ou les deux faces d'un substrat convenablement choisi en fonction de la propriété physique du matériau sensible dont les variations sont destinées à être mesurées par ce capteur.

En variante, le polymère peut également se présenter sous une forme massive comme, par exemple, un cylindre présentant une certaine porosité de sorte à rendre accessible aux composés nitrés l'ensemble des molécules formant ledit polymère.

Lorsqu'il se présente sous la forme d'un film mince, ce dernier présente, de préférence, une épaisseur de 10 angströms à 100 microns.

Un tel film peut notamment être obtenu par pulvérisation, par dépôt à la tournette ("*spin coating*" en langue anglo-saxonne) sur le substrat d'une solution contenant le polymère, ou par trempage-retrait ("*dip coating*" en langue anglo-saxonne) du substrat dans une solution contenant le polymère.

Le substrat ainsi que le système de mesure du capteur sont choisis en fonction de la propriété physique du polymère dont les variations induites par la présence de composés nitrés sont destinées à être mesurées par le capteur.

En l'espèce, les variations de masse du polymère se sont révélées particulièrement intéressantes à mesurer. Aussi, le capteur est-il, de préférence, un capteur gravimétrique.

A titre d'exemples de capteurs gravimétriques, on peut citer les capteurs du type à microbalance à quartz, les capteurs à ondes de surface, plus connus sous la terminologie anglo-saxonne "SAW" pour "Surface Acoustic Wave", tels que les capteurs à ondes de Love et les capteurs à ondes de Lamb, ainsi que les microleviers.

Parmi les capteurs gravimétriques, on préfère plus particulièrement les capteurs à microbalance à quartz. Ce type de capteurs, dont le principe de fonctionnement a été décrit par Sanchez-Pedrono et al. dans Anal. Chem. Acta, vol. 182, 1986, 285 **[4]**, comprend, schématiquement, un substrat piézo-électrique (ou résonateur), généralement un cristal de quartz recouvert sur ses deux faces d'une couche métallique, par exemple d'or ou de platine, servant d'électrode. Le matériau sensible recouvrant l'une ou les deux faces du substrat, toute variation de masse de ce matériau se traduit par une variation de la fréquence de vibration du substrat.

Bien entendu, il est également possible d'utiliser un polymère tel que précédemment défini, comme matériau sensible dans des capteurs conçus pour mesurer des variations d'une propriété physique autre que la masse comme, par exemple, des capteurs résistifs basés sur la mesure de variations de conductivité électrique ou des capteurs optiques basés sur la mesure de variations de fluorescence, de luminescence, d'absorbance dans le domaine UV-visible ou encore de longueur d'onde dans le domaine des infrarouges.

En effet, bien que les polymères comprenant un motif répétitif siloxane de formule (I) ne soient pas conducteurs de l'électricité, ils peuvent être mélangés à une ou plusieurs charges conductrices pour obtenir des composites présentant une conductivité électrique adaptée à une utilisation comme matériaux sensibles de capteurs résistifs. Ces charges conductrices peuvent être, par exemple, des particules de noir de carbone ou des poudres de métaux (Cu, Pd, Au, Pt, ...) ou d'oxydes métalliques (V₂O₃, TiO, ...).

Dans le cas d'un capteur optique, il est possible soit d'exploiter une propriété optique intrinsèque du polymère dans le cas où celui-ci en possède une (absorbance, spectre IR, ...), soit de conférer à ce polymère une propriété optique particulière par couplage avec un marqueur approprié, par exemple fluorescent ou luminescent.

Le capteur chimique selon l'invention peut être intégré dans un multicapteur, c'est-à-dire dans un dispositif réunissant plusieurs capteurs élémentaires, ces capteurs élémentaires pouvant être munis de matériaux sensibles, de substrats et/ou de systèmes de mesure différents.

Aussi, l'invention a-t-elle pour objet un multicapteur comprenant un ou plusieurs capteurs élémentaires assemblés les uns aux autres et dans lequel l'un au moins de ces capteurs élémentaires est un capteur chimique tel que précédemment défini.

Les capteurs chimiques selon l'invention se sont révélés présenter de nombreux avantages, notamment :
* une aptitude à détecter spécifiquement les composés nitrés, et en particulier les composés nitroaromatiques, avec une grande sensibilité puisqu'ils sont capables de détecter leur présence à des concentrations inférieures au ppm (partie par million) et même au dixième de ppm,
* une rapidité de réponse et une reproductibilité de cette réponse,
* une stabilité des performances dans le temps et, partant, une durée de vie très satisfaisante,
* une aptitude à fonctionner en continu,
* un coût de fabrication compatible avec une production de capteurs en série, une très faible quantité de polymère (c'est-à-dire, en pratique de quelques mg) étant nécessaire pour la fabrication d'un capteur, et
* la possibilité d'être miniaturisés et, partant, d'être aisément transportables et manipulables sur tout type de sites.

L'invention a encore pour objet l'utilisation d'un capteur chimique tel que précédemment défini, pour la détection ou le dosage d'un ou plusieurs composés nitrés, ces composés pouvant aussi bien se présenter sous forme solide, liquide ou gazeuse (vapeurs), mais étant, de préférence, sous forme gazeuse.

Conformément à l'invention, le ou les composés nitrés destinés à être détectés ou dosés sont choisis parmi les composés nitroaromatiques, les nitramines, les nitrosamines et les esters nitriques.

A titre d'exemples de composés nitroaromatiques, on peut citer le nitrobenzène, le dinitrobenzène, le trinitrobenzène, le nitrotoluène, le dinitrotoluène, le trinitrotoluène, le dinitrofluorobenzène, le dinitrotrifluorométhoxybenzène, l'aminodinitrotoluène, le dinitrotrifluorométhylbenzène, le chlorodinitrotrifluorométhylbenzène, l'hexanitrostilbène ou encore le trinitrophénol (ou acide picrique).

Les nitramines sont, elles, par exemple la cyclotétraméthylènetétranitramine (ou octogène), la cyclotriméthylènetrinitramine (ou hexogène) et la trinitrophénylméthylnitramine (ou tétryle), tandis que les nitrosamines sont, par exemple, la nitrosodiméthylamine.

Quant aux esters nitriques, il s'agit, par exemple, de pentrite, de dinitrate d'éthylène glycol, de dinitrate de diéthylène glycol, de nitroglycérine ou de nitroguanidine.

Selon encore une autre disposition préférée de l'invention, le capteur est utilisé pour la détection ou le dosage d'explosifs.

L'invention sera mieux comprise à la lumière du complément de description, qui se rapporte à des exemples de préparation de polysiloxanes anilinés aptes à servir de matériaux sensibles dans des capteurs chimiques ainsi qu'à des exemples de réalisation de capteurs chimiques comprenant de tels polysiloxanes comme matériaux sensibles et de démonstration de leurs propriétés.

Bien entendu, ces exemples ne sont donnés qu'à titre d'illustration de l'objet de l'invention et ne constituent en aucun cas une limitation de cet objet.

### BRÈVE DESCRIPTION DES DESSINS

La figure 1 représente l'évolution de la fréquence de vibration du quartz d'un premier exemple de capteur selon l'invention lorsque ce capteur est exposé à de l'air et à des vapeurs de 2,4-dinitrotrifluorométhoxybenzène (DNTFMB).
La figure 2 représente l'évolution de la fréquence de vibration du quartz d'un deuxième exemple de capteur selon l'invention lorsque ce capteur est exposé à de l'air et à des vapeurs de DNTFMB.
La figure 3 représente l'évolution de la fréquence de vibration du quartz d'un troisième exemple de capteur selon l'invention lorsque ce capteur est exposé à de l'air et à des vapeurs de DNTFMB, de toluène, de méthyléthylcétone et d'éthanol.
La figure 4 représente l'évolution de la fréquence de vibration du quartz d'un quatrième exemple de capteur selon l'invention lorsque ce capteur est exposé à de l'air et à des vapeurs de DNTFMB.
La figure 5 représente les variations de fréquence des quartz du deuxième exemple de capteur selon l'invention et d'un capteur comprenant de la N-allylaniline comme matériau sensible lorsque ces capteurs sont exposés à du DNTFMB le jour de leur élaboration (J0) et 28 jours après (J28).
La figure 6 représente l'évolution de la fréquence de vibration du quartz d'un cinquième exemple de capteur selon l'invention lorsque ce capteur est exposé à de l'air et à des vapeurs de dinitrotoluène (2,4-DNT).

### EXPOSÉ DÉTAILLÉ DE MODES DE REALISATION PARTICULIERS

### Exemple 1 : Préparation de polysiloxanes anilinés

On prépare quatre polymères, désignés respectivement ci-après A, B, C et D, et présentant les compositions suivantes :
Polymère A :
   homopolymère de motif répétitif de formule (Ia) ;
Polymère B :
   copolymère aléatoire formé d'un motif répétitif de formule (Ia) et d'un motif répétitif diméthylsiloxane dans un rapport de 0,47 motif de formule (Ia) pour 0,53 motif diméthylsiloxane ;
Polymère C :
   copolymère aléatoire formé d'un motif répétitif de formule (Ib) et d'un motif répétitif diméthylsiloxane dans un rapport de 0,47 motif de formule (Ib) pour 0,53 motif diméthylsiloxane ;
Polymère D :
   copolymère aléatoire formé d'un motif répétitif de formule (Ic) et d'un motif répétitif diméthylsiloxane dans un rapport de 0,47 motif de formule (Ic) pour 0,53 motif diméthylsiloxane ;
en modifiant des polysiloxanes comprenant, dans le cas de la préparation du polymère A, uniquement un motif répétitif méthylhydrogénosiloxane de formule (IV) ci-après : 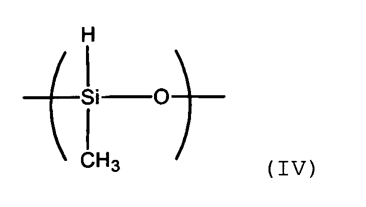 et, dans le cas de la préparation des polymères B, C et D, un motif répétitif méthylhydrogénosiloxane et un motif répétitif diméthylsiloxane dans un rapport de 0,47 motif méthylhydrogénosiloxane pour 0,53 motif diméthylsiloxane.

Ces polysiloxanes sont notamment disponibles auprès de la société ABCR sous les références HMS 992 et HMS 501.

La modification consiste en un couplage d'un composé aminé allylique de formule (V) ci-après : 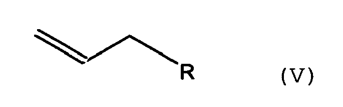 dans laquelle R représente l'un des groupes suivants : 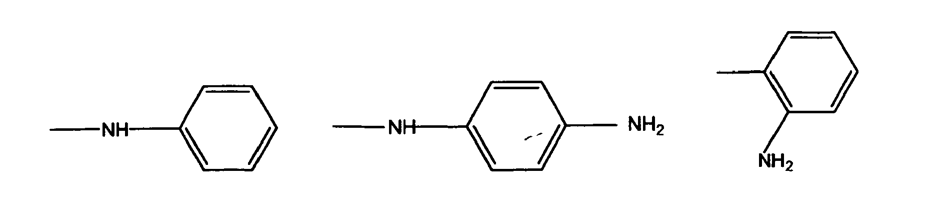 sur le motif répétitif de formule (IV) par hydrosilylation catalytique selon le schéma réactionnel suivant :

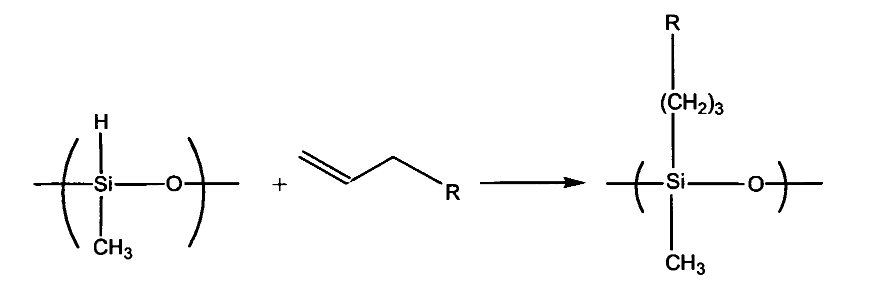

Cette hydrosilylation est réalisée en masse pour les polymères A et B, et en solution pour les polymères C et D.

Dans le cas des polymères A et B, le monomère allylique (1,5 mmole de N-allylaniline, disponible chez Sigma-Aldrich sous la référence A2,900-3) est chauffé à 80°C sous un courant d'azote. Le catalyseur (25 µl d'une solution d'acide hexachloro-platinique (H₂PtCl₆), disponible chez Sigma-Aldrich sous la référence 52,089-6, dans l'isopropanol à 0,84% en masse ou 20 µl de catalyseur de Karstedt, disponible chez Sigma-Aldrich sous la référence 47,951-9) est ajouté. Après 15 minutes d'agitation, le polysiloxane est ajouté goutte à goutte et la solution est chauffée à 105°C pendant 5 heures. Après filtration sur du charbon actif, le produit est purifié en éliminant les volatils par distillation sous vide.

On obtient ainsi les polymères A et B sous forme d'huiles oranges avec un rendement respectivement de 96% et 93%.

Dans le cas des polymères C et D, le monomère allylique (1,5 mmole de 4-amino-N-allylaniline, synthétisée comme décrit par Kaufman et al., Russian J. Org. Chem., vol. 37, 5, 2001, 707, **[5],** pour le polymère C, et 1,5 mmole de 2-allylaniline, synthétisée comme décrit par Tsuji et al., Chem. Lett., 1995, 1121-1122, **[6],** pour le polymère D) en solution dans 3 ml de toluène est chauffé à 80°C sous un courant d'azote. Le catalyseur (0,84% en masse de H₂PtCl₆ en solution dans l'isopropanol ou 20 µl de catalyseur de Karstedt) est ajouté. Après 15 minutes d'agitation, le polysiloxane est ajouté goutte à goutte et la solution est chauffée à 105°C pendant 5 heures. Après filtration sur du charbon actif, le produit est purifié en éliminant les volatils par distillation sous vide.

On obtient ainsi le polymère C sous forme d'une huile noire avec un rendement de 28%, et le polymère D sous forme d'une huile marron avec un rendement de 84%.

### Exemple 2 : Détection du DNTFMB, par un capteur selon l'invention

Dans cet exemple, on réalise un capteur à microbalance à quartz en recouvrant les deux faces d'un quartz de coupe AT, de fréquence de vibration de 9 MHz, muni de deux électrodes de mesure circulaires en or (modèle QA9RA-50, AMETEK PRECISION INSTRUMENTS), d'un film mince du polymère B préparé conformément à l'exemple 1.

Le dépôt de ce film est réalisé en effectuant sur chaque face du quartz 20 pulvérisations de 0,3 seconde chacune d'une solution du polymère B dans le chloroforme, de concentration égale à 5 g/L.

La variation de la fréquence de vibration du quartz due à ce dépôt est de 9,9 kHz.

Le capteur est exposé successivement à :
- de l'air pendant 2100 secondes,
- du DNTFMB à une concentration de 3 ppm dans de l'air pendant 600 secondes, et
- de l'air pendant 2310 secondes,
l'air et le DNTFMB étant à température ambiante.

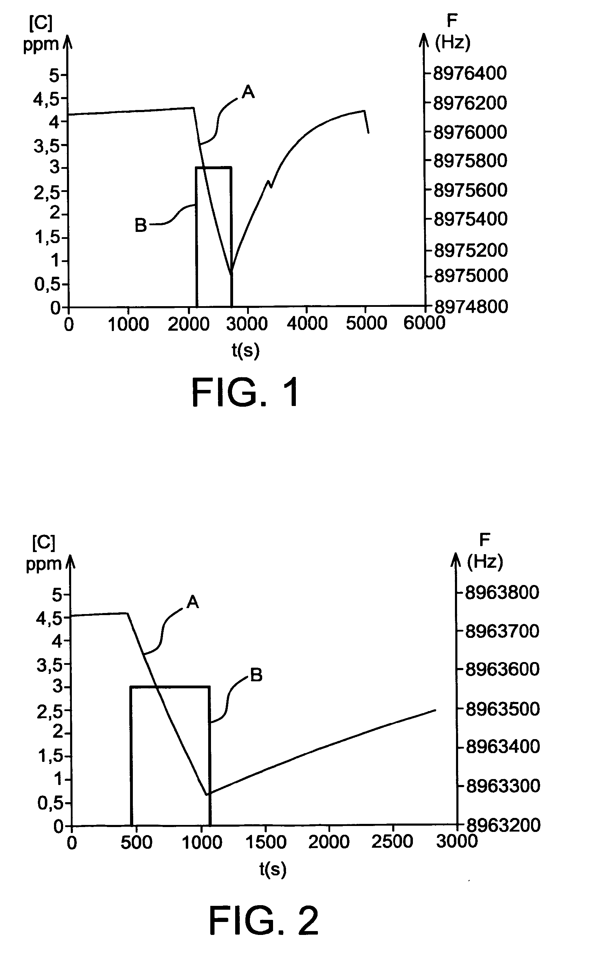

La figure 1 illustre l'évolution de la fréquence de vibration du quartz au cours de ces expositions.

Sur cette figure, la courbe A représente les valeurs de la fréquence de vibration (F) du quartz, exprimées en Hz (hertz), en fonction du temps (t), exprimé en secondes, tandis que la courbe B représente les valeurs de la concentration en DNTFMB ([C]), exprimées en ppm, également en fonction du temps.

### Exemple 3 : Détection du DNTFMB par un capteur selon l'invention

Dans cet exemple, on réalise un capteur à microbalance à quartz en recouvrant les deux faces d'un quartz identique à celui utilisé dans l'exemple 2 d'un film mince du polymère A préparé conformément à l'exemple 1.

Le dépôt de ce film est réalisé en effectuant sur chaque face du quartz 8 pulvérisations de 0,3 seconde chacune d'une solution du polymère A dans le chloroforme, de concentration égale à 10 g/L.

La variation de la fréquence de vibration du quartz due à ce dépôt est de 9,5 kHz.

Le capteur est exposé successivement à :
- de l'air pendant 460 secondes,
- du DNTFMB à une concentration de 3 ppm dans de l'air pendant 600 secondes, et
- de l'air pendant 1800 secondes,
l'air et le DNTFMB étant à température ambiante.

La figure 2 illustre l'évolution de la fréquence de vibration du quartz au cours de ces expositions.

Sur cette figure, la courbe A représente les valeurs de la fréquence de vibration (F) du quartz, exprimées en Hz, en fonction du temps (t), exprimé en secondes, tandis que la courbe B représente les valeurs de la concentration en DNTFMB ([C]), exprimées en ppm, également en fonction du temps.

### Exemple 4 : Mise en évidence de la sélectivité d'un capteur selon l'invention pour des composés nitrés vis-à-vis des solvants

Dans cet exemple, on réalise un capteur à microbalance à quartz en recouvrant les deux faces d'un quartz identique à celui utilisé dans l'exemple 2 d'un film mince du polymère C préparé conformément à l'exemple 1.

Le dépôt de ce film est réalisé en effectuant sur chaque face du quartz 23 pulvérisations de 0,4 seconde chacune d'une solution du polymère C dans le chloroforme, de concentration égale à 5 g/L.

La variation de la fréquence de vibration du quartz due à ce dépôt est de 10 kHz.

Le capteur est exposé successivement à :
- de l'air pendant 1550 secondes,
- du DNTFMB à une concentration de 3 ppm dans de l'air pendant 600 secondes,
- de l'air pendant 1820 secondes,
- du DNTFMB à une concentration de 3 ppm dans de l'air pendant 600 secondes,
- de l'air pendant 1650 secondes,
- du toluène à une concentration de 38 000 ppm dans de l'air pendant 600 secondes,
- de l'air pendant 130 secondes,
- de la méthyléthylcétone à une concentration de 126 000 ppm dans l'air pendant 600 secondes,
- de l'air pendant 170 secondes,
- de l'éthanol à une concentration de 79 000 ppm dans de l'air pendant 170 secondes, et à
- de l'air pendant 50 secondes,
l'air, le DNTFMB, le toluène, la méthyléthylcétone et l'éthanol étant à température ambiante.

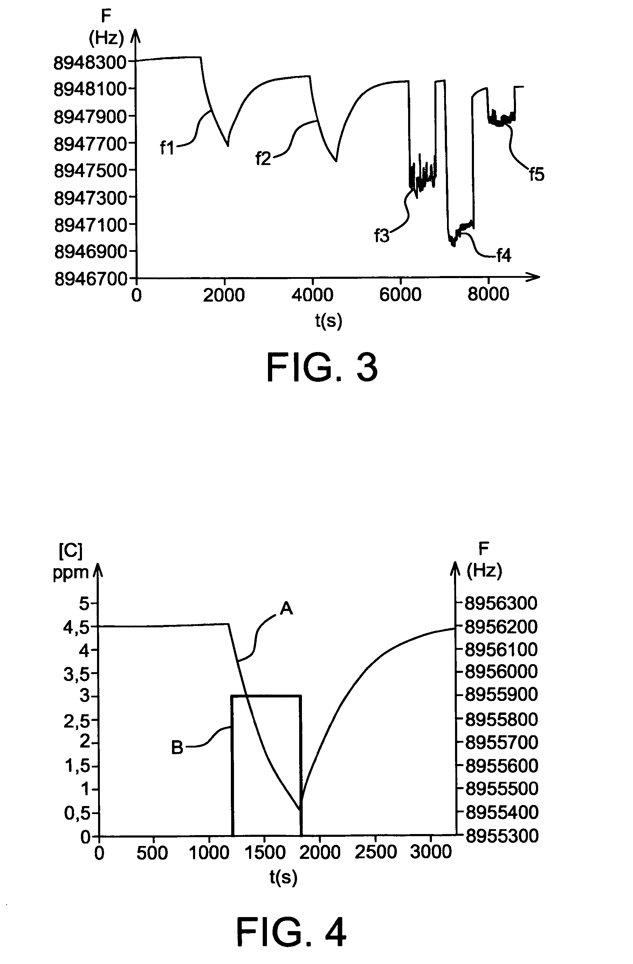

La figure 3 représente les valeurs de la fréquence de vibration (F) du quartz, exprimées en Hz, en fonction du temps (t), exprimé en secondes, les flèches f1 et f2 signalant les expositions au DNTFMB, la flèche f3 l'exposition au toluène, la flèche f4 l'exposition à la méthyléthylcétone et la flèche f5 celle à l'éthanol.

Cette figure montre que l'exposition du capteur selon l'invention à des solvants tels que le toluène, la méthyléthylcétone ou l'éthanol, ne provoque pas une réponse du capteur comparable à celle obtenue lorsque ce dernier est exposé à un composé nitré.

### Exemple 5 : Détection du DNTFMB par un capteur selon l'invention

Dans cet exemple, on réalise un capteur à microbalance à quartz en recouvrant les deux faces d'un quartz identique à celui utilisé dans l'exemple 2 d'un film mince du polymère D préparé conformément à l'exemple 1.

Le dépôt de ce film est réalisé en effectuant sur chaque face du quartz 16 pulvérisations de 0,3 seconde chacune d'une solution du polymère D dans le chloroforme, de concentration égale à 5 g/L.

La variation de la fréquence de vibration du quartz due à ce dépôt est de 10 kHz.

Le capteur est exposé successivement à :
- de l'air pendant 1224 secondes,
- du DNTFMB à une concentration de 3 ppm dans de l'air pendant 600 secondes,
- de l'air pendant 1480 secondes,
l'air et le DNTFMB étant à température ambiante.

La figure 4 illustre l'évolution de la fréquence de vibration du quartz au cours de ces expositions.

Sur cette figure, la courbe A représente les valeurs de la fréquence de vibration (F) du quartz, exprimées en Hz, en fonction du temps (t), exprimé en secondes, tandis que la courbe B représente les valeurs de la concentration en DNTFMB ([C]), exprimées en ppm, également en fonction du temps.

### Exemple 6 : Mise en évidence de la stabilité des performances d'un capteur selon l'invention et d'un capteur comprenant de la N-allylaniline comme matériau sensible

Dans cet exemple, on compare les performances du capteur utilisé dans l'exemple 3 avec celles d'un capteur de structure identique mais comprenant, sur chaque face, un film de N-allylaniline en lieu et place du film de polymère B.

Le dépôt du film de N-allylaniline est réalisé en effectuant sur chaque face du quartz 12 pulvérisations de 0,5 seconde chacune d'une solution de ce composé dans le chloroforme, de concentration égale à 10 g/L, de sorte à conduire à une variation de la fréquence de vibration du quartz de 2 kHz.

Les deux capteurs sont exposés pendant 10 minutes à du DNTFMB, à une concentration de 3 ppm dans de l'air, le jour de leur élaboration (J0) et 28 jours après (J28). Entre les deux expositions, ils sont conservés à l'air ambiant sans conditions particulières.

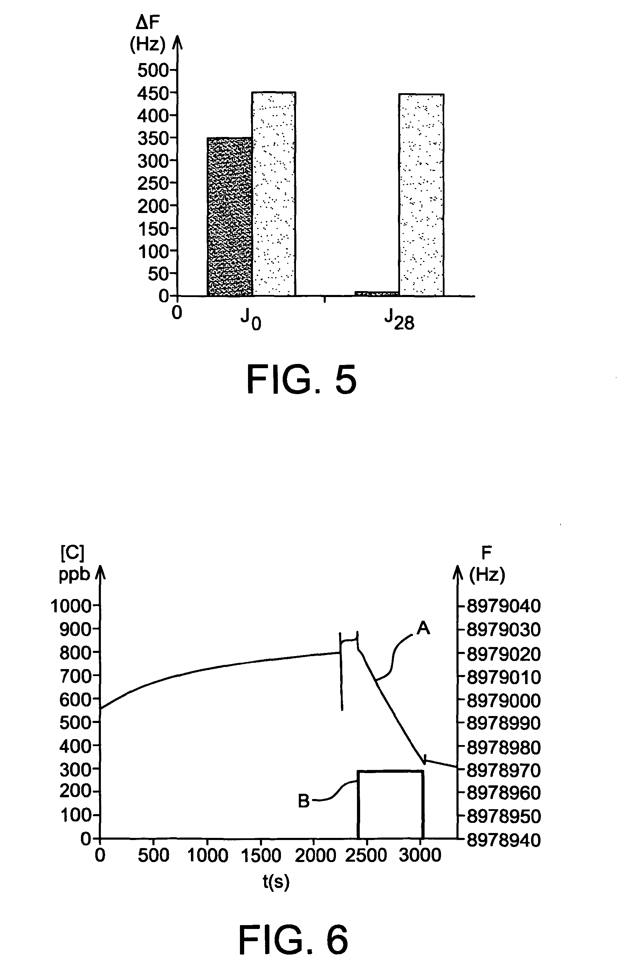

La figure 5 montre les variations de fréquence du quartz (ΔF), exprimées en Hz, obtenues pour les deux capteurs à J0 et J28, celles correspondant au capteur selon l'invention étant représentées en grisé clair et celles correspondant au capteur comprenant de la N-allylaniline comme matériau sensible étant représentées en grisé foncé.

### Exemple 7 : Détection du 2,4-DNT par un capteur selon l'invention

Dans cet exemple, on réalise un capteur à microbalance à quartz en recouvrant les deux faces d'un quartz identique à celui utilisé dans l'exemple 2 d'un film mince du polymère B préparé conformément à l'exemple 1.

Le dépôt de ce film est réalisé en effectuant sur chaque face du quartz 17 pulvérisations de 0,3 seconde chacune d'une solution du polymère B dans le chloroforme, de concentration égale à 10 g/L.

La variation de la fréquence de vibration du quartz due à ce dépôt est de 9,9 kHz.

Le capteur est exposé successivement à :
- de l'air pendant 2450 secondes,
- du 2,4-DNT à une concentration de 285 ppb dans de l'air pendant 600 secondes, et
- de l'air pendant 350 secondes,
l'air et le 2,4-DNT étant à température ambiante.

La figure 5 illustre l'évolution de la fréquence de vibration du quartz au cours de ces expositions.

Sur cette figure, la courbe A représente les valeurs de la fréquence de vibration (F) du quartz, exprimées en Hz, en fonction du temps (t), exprimé en secondes, tandis que la courbe B représente les valeurs de la concentration en 2,4-DNT ([C]), exprimées en ppb, également en fonction du temps.

### Exemple 8 : Comparaison des performances d'un capteur selon l'invention et d'un capteur comprenant un film mince d'un polysiloxane selon McGill et al (ibid).

Dans cet exemple, on réalise deux capteurs à microbalance à quartz comprenant tous deux un quartz identique à celui utilisé dans l'exemple 2, mais se différenciant l'un de l'autre en ce que le quartz du premier est recouvert sur ses deux faces d'un film mince du polymère B, alors que le quartz du second est recouvert d'un film mince d'un polysiloxane comportant des motifs fonctionnalisés par un groupe phényle substitué par deux groupes HFIP.

Ce polysiloxane répond à la formule (VI) ci-après :

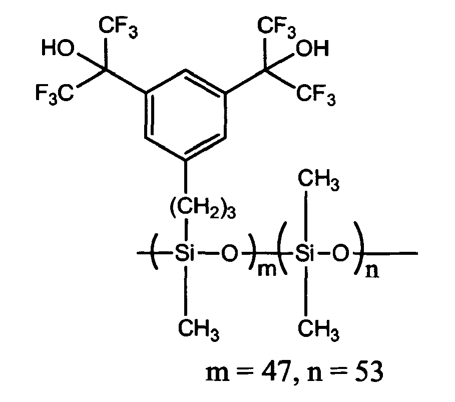

Les dépôts des films sont réalisés de sorte que la variation de la fréquence de vibration des quartz due à ces dépôts soit égale à 10 kHz pour chacun des capteurs.

Pour ce faire, le dépôt du film de polymère B est effectué en pulvérisant 20 fois 0,3 seconde une solution du polymère B dans du chloroforme, de concentration égale à 5 g/L, sur les deux faces du capteur, tandis que le dépôt du film du polysiloxane de formule (VI) est effectué en pulvérisant 30 fois 0,2 seconde une solution dudit polysiloxane dans du dichlorométhane, de concentration égale à 2 g/L, sur les deux faces du quartz.

Les deux capteurs sont exposés, exactement dans les mêmes conditions, à des vapeurs de DNTFMB de concentration égale à 3 ppm, à température ambiante et pendant 10 minutes.

La mesure de la fréquence de vibration du quartz des deux capteurs au temps t₀ et au temps t₁₀ₘᵢₙ de cette exposition donne une variation de la fréquence de vibration de 1 100 Hz pour le quartz du capteur selon l'invention, et de 490 Hz - soit plus de 2 fois plus faible - pour le quartz du capteur comprenant le film mince du polysiloxane de formule (VI).

### REFERENCES CITEES

**[1]** Briglin et al., Proceedings of SPIE, vol. 4394, 2001, 912-921,
**[2]** McGill et al., Sens. Actuators B65, 2000, 5-9,
**[3]** PCT WO-A-02/08314
**[4]** Sanchez-Pedrono et al., Anal. Chem. Acta, vol. 182, 1986, 285
**[5]** Kaufman et al., Russian J. Org. Chem., vol. 37, 5, 2001, 707
**[6]** Tsuji et al., Chem. Lett., 1995, 1121-1122

## Revendications

1. Capteur chimique comprenant, en tant que matériau sensible, au moins un polymère comprenant un motif répétitif siloxane qui répond à la formule (I) ci-après : 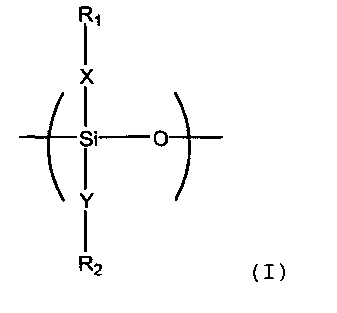 dans laquelle :
- X et Y, qui peuvent être identiques ou différents, représentent une liaison simple ou un groupe hydrocarboné linéaire, ramifié ou cyclique, saturé ou insaturé, comprenant de 1 à 30 atomes de carbone et éventuellement un ou plusieurs hétéroatomes et/ou une ou plusieurs fonctions chimiques comportant au moins un hétéroatome et/ou un ou plusieurs groupes aromatiques ou hétéroaromatiques ;
- R₁ représente un groupe aniline répondant à la formule (II) ci-après : 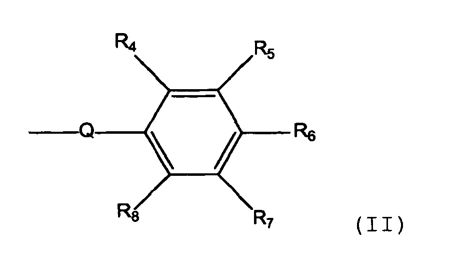 dans laquelle :
• Q représente une liaison simple, un groupe -CH₂-, ou bien un groupe -NR₃- dans lequel R₃ représente un atome d'hydrogène ou un groupe hydrocarboné linéaire, saturé ou insaturé, et comprenant de 1 à 10 atomes de carbone ;
• R₄ à R₈, qui peuvent être identiques ou différents, représentent un atome d'hydrogène, une fonction chimique comportant au moins un hétéroatome, ou bien un groupe hydrocarboné linéaire, ramifié ou cyclique, saturé ou insaturé, comprenant de 1 à 30 atomes de carbone et éventuellement un ou plusieurs hétéroatomes et/ou une ou plusieurs fonctions chimiques comportant au moins un hétéroatome et/ou un ou plusieurs groupes aromatiques ou hétéro-aromatiques ;
et dans laquelle, lorsque Q est une liaison simple ou un groupe -CH₂-, alors au moins l'un des radicaux R₄ à R₈ représente un groupe aminé -NR₉R10 dans lequel R₉ et R₁₀ représentent, indépendamment l'un de l'autre, un atome d'hydrogène, une fonction chimique comportant au moins un hétéroatome, ou bien un groupe hydrocarboné linéaire, ramifié ou cyclique, saturé ou insaturé, comprenant de 1 à 30 atomes de carbone et éventuellement un ou plusieurs hétéroatomes et/ou une ou plusieurs fonctions chimiques comportant au moins un hétéroatome et/ou un ou plusieurs groupes aromatiques ou hétéro-aromatiques ;
- R₂ représente un atome d'hydrogène ou un groupe aniline de formule (II) telle que définie ci-dessus.

2. Capteur selon la revendication 1, dans lequel le motif répétitif siloxane répond à la formule (I) dans laquelle R₁ représente un groupe de formule (IIa), (IIb) ou (IIc) ci-après :

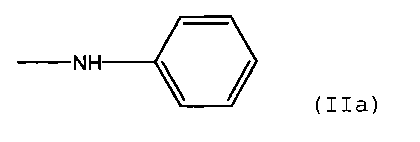

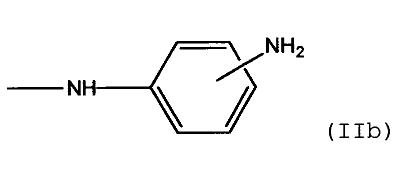

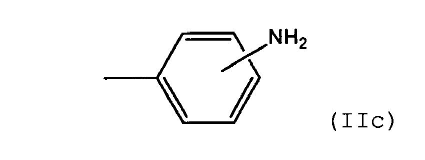

3. Capteur selon la revendication 1 ou la revendication 2, dans lequel le motif répétitif siloxane répond à la formule (I) dans laquelle X représente un groupe alkylène comprenant de 1 à 10 atomes de carbone, Y représente un groupe alkylène comprenant de 1 à 3 atomes de carbone tandis que R₂ représente un atome d'hydrogène.

4. Capteur selon la revendication 3, dans lequel le motif répétitif siloxane répond à la formule (I) dans laquelle X représente un groupe propylène, Y représente un groupe méthylène tandis que R₂ représente un atome d'hydrogène.

5. Capteur selon l'une quelconque des revendications précédentes, dans lequel le motif répétitif siloxane répond à l'une des formules (Ia), (Ib) et (Ic) ci-après :

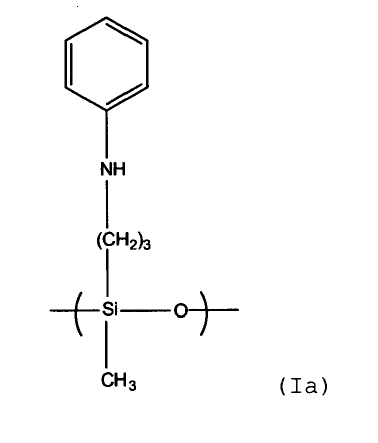

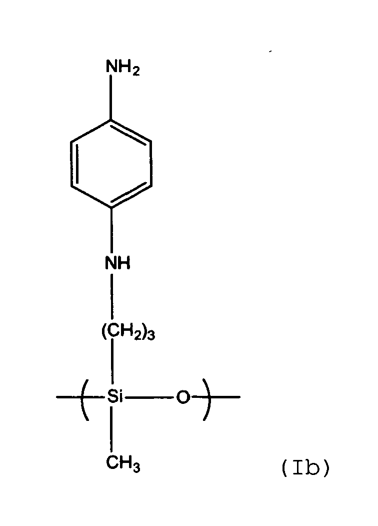

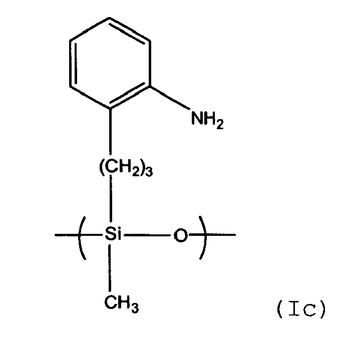

6. Capteur selon l'une quelconque des revendications précédentes, dans lequel le polymère est un homopolymère.

7. Capteur selon l'une quelconque des revendications 1 à 5, dans lequel le polymère est un copolymère qui comprend deux motifs répétitifs siloxane différents, un premier motif répondant à la formule (I) dans laquelle X, Y, R₁ et R₂ ont la même signification que précédemment, et un deuxième motif répondant à la formule (III) ci-après : 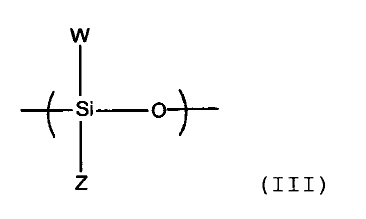 dans laquelle W et Z, qui peuvent être identiques ou différents, représentent un atome d'hydrogène, un groupe hydrocarboné, linéaire, ramifié ou cyclique, saturé ou insaturé, comprenant de 1 à 30 atomes de carbone.

8. Capteur selon la revendication 7, dans lequel le deuxième motif siloxane est un motif dihydrogénosiloxane, un motif méthylhydrogénosiloxane ou un motif diméthylsiloxane.

9. Capteur selon la revendication 6, dans lequel le polymère est un homopolymère constitué par le motif répétitif siloxane de formule (Ia) ci-après :

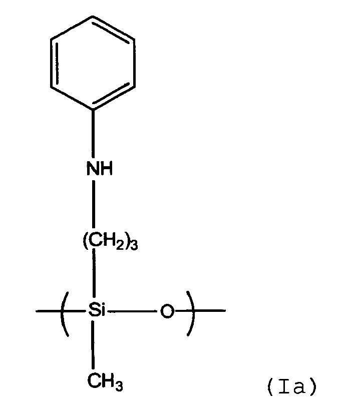

10. Capteur selon la revendication 8, dans lequel le polymère est un copolymère qui comprend un premier motif siloxane répondant à la formule (Ia) ci-après : 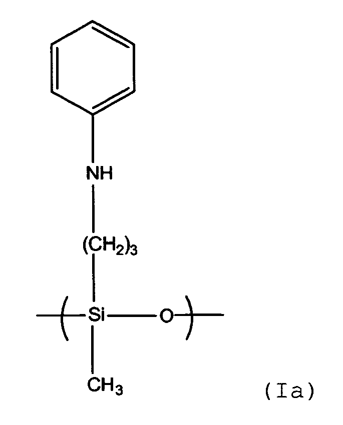 et un deuxième motif diméthylsiloxane.

11. Capteur selon la revendication 8, dans lequel le polymère est un copolymère qui comprend un premier motif siloxane répondant à la formule (Ib) ci-après : 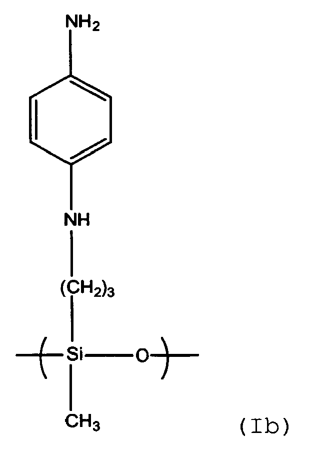 et un deuxième motif diméthylsiloxane.

12. Capteur selon la revendication 8, dans lequel le polymère est un copolymère qui comprend un premier motif siloxane répondant à la formule (Ic) ci-après : 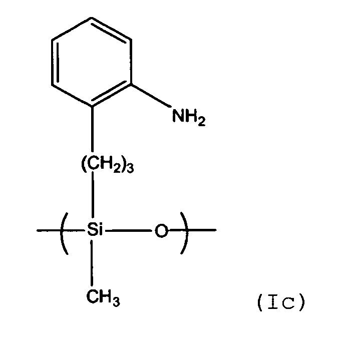 et un deuxième motif diméthylsiloxane.

13. Capteur selon l'une quelconque des revendications 10 à 12, dans lequel le copolymère est aléatoire, alterné ou séquencé.

14. Capteur selon l'une quelconque des revendications 10 à 11, dans lequel le premier motif répétitif représente, en nombre, environ 47% des motifs répétitifs formant le copolymère, tandis que le deuxième motif répétitif représente, en nombre, environ 53% des motifs formant le copolymère.

15. Capteur selon l'une quelconque des revendications précédentes, dans lequel le polymère ou le composite est présent sous la forme d'un film mince recouvrant l'une ou les deux faces d'un substrat.

16. Capteur selon la revendication 15, dans lequel le film mince mesure de 10 angströms à 100 microns d'épaisseur.

17. Capteur selon l'une quelconque des revendications précédentes, qui est un capteur est un capteur gravimétrique.

18. Capteur selon l'une quelconque des revendications précédentes, qui est un capteur à microbalance à quartz.

19. Multicapteur comprenant un ou plusieurs capteurs élémentaires assemblés les uns aux autres et dans lequel l'un au moins de ces capteurs élémentaires est un capteur chimique selon l'une quelconque des revendications 1 à 18.

20. Utilisation d'un capteur chimique selon l'une quelconque des revendications 1 à 18 ou d'un multicapteur selon la revendication 19, pour la détection ou le dosage d'un ou plusieurs composés nitrés.

21. Utilisation selon la revendication 20, dans laquelle le ou les composés nitrés sont choisis parmi les composés nitroaromatiques, les nitramines, les nitrosamines et les esters nitriques.

22. Utilisation selon la revendication 21, dans laquelle le ou les composés nitrés à détecter sont sous forme gazeuse.

23. Utilisation selon l'une quelconque des revendications 20 à 22, dans laquelle le ou les composés nitrés sont choisis parmi le nitrobenzène, le dinitrobenzène, le trinitrobenzène, le nitrotoluène, le dinitrotoluène, le trinitrotoluène, le dinitrofluorobenzène, le dinitrotrifluorométhoxybenzène, l'aminodinitrotoluène, le dinitrotrifluorométhylbenzène, le chlorodinitrotrifluorométhylbenzène, l'hexanitrostilbène, le trinitrophénol, la cyclotétraméthylènetétranitramine, la cyclotriméthylènetrinitramine, la trinitrophénylméthylnitramine, la nitrosodiméthylamine, le pentrite, le dinitrate d'éthylène glycol, le dinitrate de diéthylène glycol, la nitroglycérine ou la nitroguanidine.

24. Utilisation selon l'une quelconque des revendications 20 à 23, pour la détection d'explosifs.

## Claims

1. Chemical sensor comprising, as sensitive material, at least one polymer comprising a siloxane repeating unit which corresponds to formula (I) below: 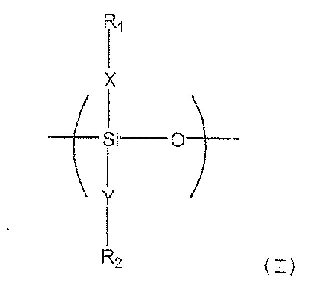 in which:
- X and Y, which may be identical or different, represent a single bond or a saturated or unsaturated, linear, branched or cyclic hydrocarbon group comprising from 1 to 30 carbon atoms and, optionally, one or more heteroatoms, and/or one or more chemical functions containing at least one heteroatom, and/or one or more aromatic or heteroaromatic groups;
- R₁ represents an aniline group corresponding to formula (II) bellow: 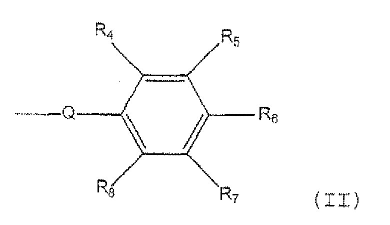 in which:
■ Q represents a single bond, a -CH₂- group, or else an -NR₃- group in which R₃ represents a hydrogen atom or a saturated or unsaturated, linear hydrocarbon group comprising from 1 to 10 carbon atoms;
■ R₄ to R₈ represent, independently of one another, a hydrogen atom, a chemical function containing at least one heteroatom, or else a saturated or unsaturated, linear, branched or cyclic hydrocarbon group comprising from 1 to 30 carbon atoms and, optionally, one or more heteroatoms, and/or one or more chemical functions containing at least one heteroatom, and/or one or more aromatic or heteroaromatic groups;
and in which, when Q is a single bond or a -CH₂- group, then at least one of the radicals R₄ to R₈ represents an amino group -NR₉R₁₀ in which R₉ and R₁₀ represent, independently of one another, a hydrogen atom, a chemical function containing at least one heteroatom, or else a saturated or unsaturated, linear, branched or cyclic hydrocarbon group comprising from 1 to 30 carbon atoms and, optionally, one or more heteroatoms, and/or one or more chemical functions containing at least one heteroatom, and/or one or more aromatic or heteroaromatic groups;
- R₂ represents a hydrogen atom or an aniline group of formula (II) as defined above.

2. Sensor according to Claim 1, in which the siloxane repeating unit corresponds to formula (I) in which R₁ represents a group of formula (IIa), (IIb) or (IIc) below:

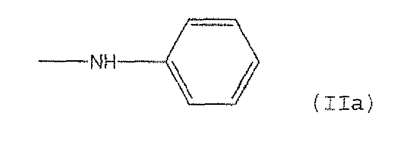

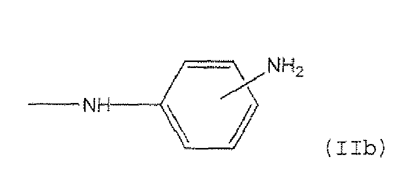

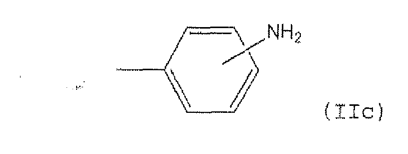

3. Sensor according to Claim 1 or Claim 2, in which the siloxane repeating unit corresponds to formula (I) in which X represents an alkylene group comprising from 1 to 10 carbon atoms, Y represents an alkylene group containing from 1 to 3 carbon atoms, while R₂ represents a hydrogen atom.

4. Sensor according to Claim 3, in which the siloxane repeating unit corresponds to formula (I) in which X represents a propylene group, Y represents a methylene group, while R₂ represents a hydrogen atom.

5. Sensor according to any one of the preceding claims, in which the siloxane repeating unit corresponds to one of formulae (Ia), (Ib) and (Ic) below:

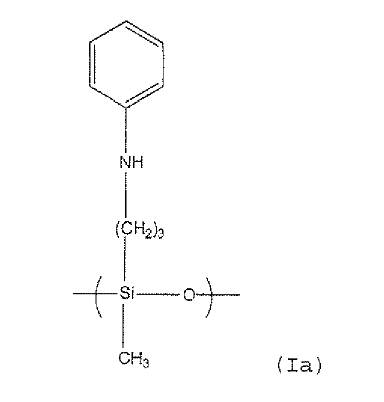

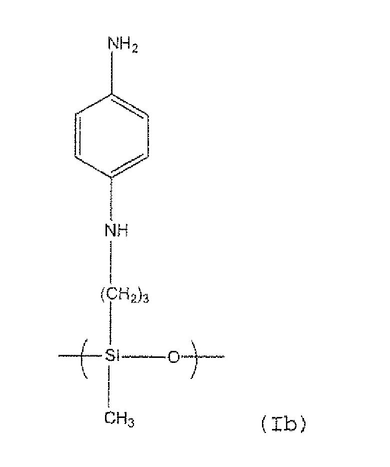

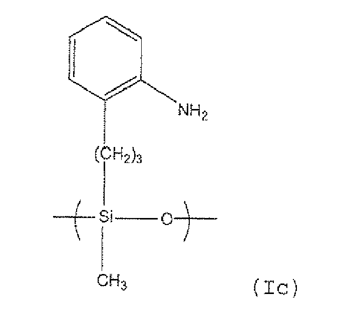

6. Sensor according to any one of the preceding claims, in which the polymer is a homopolymer.

7. Sensor according to any one of Claims 1 to 5, in which the polymer is a copolymer which comprises two different siloxane repeating units, a first unit corresponding to formula (I) in which X, Y, R₁ and R₂ have the same meaning as above, and a second unit corresponding to formula (III) below: 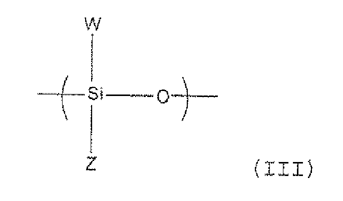 in which W and Z, which may be identical or different, represent a hydrogen atom, or a saturated or unsaturated, linear, branched or cyclic hydrocarbon group comprising from 1 to 30 carbon atoms.

8. Sensor according to Claim 7, in which the second siloxane unit is a dihydrosiloxane unit, a methyl-hydrosiloxane unit or a dimethylsiloxane unit.

9. Sensor according to Claim 6, in which the polymer is a homopolymer consisting of the siloxane repeating unit of formula (Ia) below:

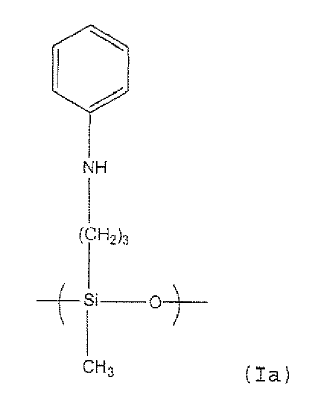

10. Sensor according to Claim 8, in which the polymer is a copolymer which comprises a first siloxane unit corresponding to formula (Ia) below: 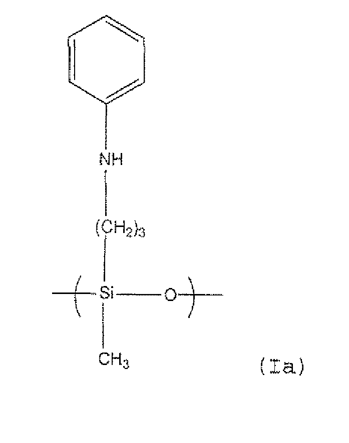 and a second dimethylsiloxane unit.

11. Sensor according to Claim 8, in which the polymer is a copolymer which comprises a first siloxane unit corresponding to formula (Ib) below: 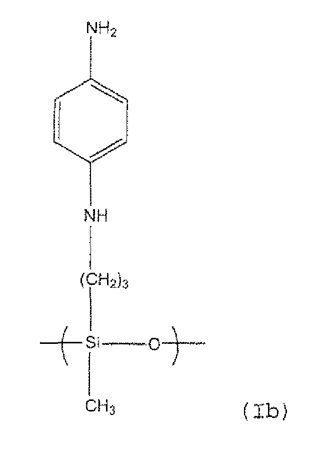 and a second dimethylsiloxane unit.

12. Sensor according to Claim 8, in which the polymer is a copolymer which comprises a first siloxane unit corresponding to formula (Ic) below: 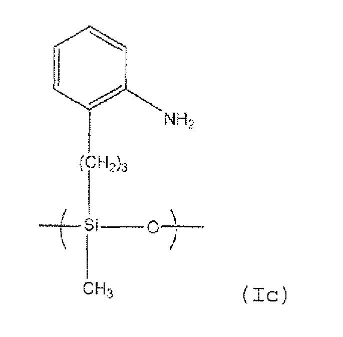 and a second dimethylsiloxane unit.

13. Sensor according to any one of Claims 10 to 12, in which the copolymer is a random, alternating or block copolymer.

14. Sensor according to either one of claims 10 and 11, in which the first repeating unit represents, by number, approximately 47% of the repeating units forming the copolymer, while the second repeating unit represents, by number, approximately 53% of the units forming the copolymer.

15. Sensor according to any one of the preceding claims, in which the polymer or the composite is present in the form of a thin film covering one or both faces of a substrate.

16. Sensor according to Claim 15, in which the thin film is 10 angstroms to 100 microns thick.

17. Sensor according to any one of the preceding claims, which is a gravimetric sensor.

18. Sensor according to any one of the preceding claims, which is a quartz microbalance sensor.

19. Multisensor comprising one or more individual sensors assembled together and in which at least one of these individual sensors is a chemical sensor according to any one of Claims to 18.

20. Use of a chemical sensor according to any one of Claims 1 to 18 or of a multisensor according to Claim 19, for detecting or assaying one or more nitro compounds.

21. Use according to Claim 20, in which the nitro compound(s) is (are) chosen from nitroaromatic compounds, nitramines, nitrosamines and nitric esters.

22. Use according to Claim 21, in which the nitro compound(s) to be detected is (are) in gaseous form.

23. Use according to any one of Claims 20 to 22, in which the nitro compound(s) is (are) chosen from nitrobenzene, dinitrobenzene, trinitrobenzene, nitrotoluene, dinitrotoluene, trinitrotoluene, dinitrofluorobenzene, dinitrotrifluoromethoxybenzene, aminodinitrotoluene, dinitrotrifluoromethylbenzene, chlorodinitrotrifluoromethylbenzene, hexanitrostilbene, trinitrophenol, cyclotetramethylenetetranitramine, cyclotrimethylenetrinitramine, trinitrophenylmethylnitramine, nitrosodimethylamine, pentrite, ethylene glycol dinitrate, diethylene glycol dinitrate, nitroglycerine or nitroguanidine.

24. Use according to any one of Claims 20 to 23, for detecting explosives.

## Patentansprüche

1. Chemischer Sensor, der als empfindliches Material wenigstens ein Polymer umfasst, das eine der nachstehenden Formel (I) entsprechende, sich wiederholende Siloxaneinheit umfasst: 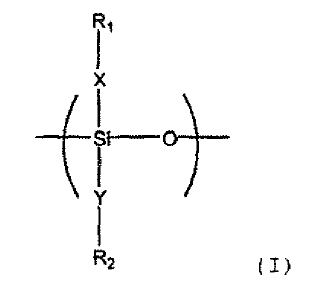 worin:
- X und Y, die gleich oder verschieden sein können, eine Einfachbindung oder eine gesättigte oder ungesättigte, gerade, verzweigte oder cyclische Kohlenwasserstoffgruppe darstellen, die 1 bis 30 Kohlenstoffatome und gegebenenfalls ein oder mehrere Heteroatome und/oder eine oder mehrere chemische Funktionen umfasst, die wenigstens ein Heteroatom und/oder eine oder mehrere aromatische oder heteroaromatische Gruppen aufweisen,
- R₁ eine der nachstehenden Formel (II) entsprechende Anilingruppe darstellt: 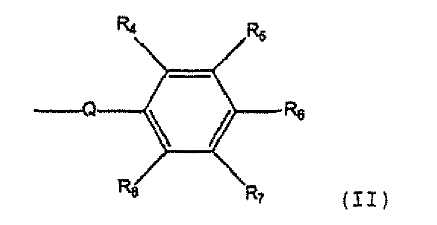 worin:
• Q eine Einfachbindung, eine -CH₂-Gruppe oder eine Gruppe -NR₃- darstellt, worin R₃ ein Wasserstoffatom oder eine gesättigte oder ungesättigte, gerade Kohlenwasserstoffgruppe darstellt und 1 bis 10 Kohlenstoffatome umfasst,
• R₄ bis R₈, die gleich oder verschieden sein können, ein Wasserstoffatom, eine chemische Funktion, die wenigstens ein Heteroatom aufweist, oder eine gesättigte oder ungesättigte, gerade, verzweigte oder cyclische Kohlenwasserstoffgruppe darstellen, die bis 30 Kohlenstoffatome und gegebenenfalls ein oder mehrere Heteroatome und/oder eine oder mehrere chemische Funktionen umfasst, die wenigstens ein Heteroatom und/oder eine oder mehrere aromatische oder heteroaromatische Gruppen aufweisen,
und worin dann, wenn Q eine Einfachbindung oder eine -CH₂-Gruppe ist, wenigstens einer der Reste R₄ bis R₈ eine Amingruppe -NR₉R₁₀ darstellt, worin R₉ und R₁₀ unabhängig voneinander ein Wasserstoffatom, eine chemische Funktion, die wenigstens ein Heteroatom aufweist, oder eine gesättigte oder ungesättigte, gerade, verzweigte oder cyclische Kohlenwasserstoffgruppe darstellen, die 1 bis 30 Kohlenstoffatome und gegebenenfalls ein oder mehrere Heteroatome und/oder eine oder mehrere chemische Funktionen umfasst, die wenigstens ein Heteroatom und/oder eine oder mehrere aromatische oder heteroaromatische Gruppen aufweisen, und
- R₂ ein Wasserstoffatom oder eine vorstehend definierte Anilingruppe der Formel (II) darstellt.

2. Sensor gemäß Anspruch 1, bei dem die sich wiederholende Siloxaneinheit der Formel (I) entspricht, worin R₁ eine Gruppe der nachstehenden Formel (IIa), (IIb) oder (IIc) darstellt:

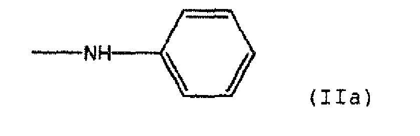

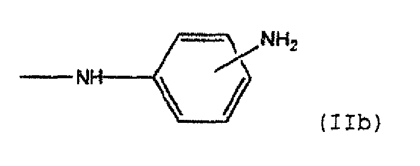

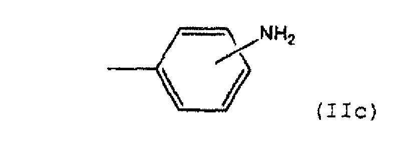

3. Sensor gemäß Anspruch 1 oder Anspruch 2, bei dem die sich wiederholende Siloxaneinheit der Formel (I) entspricht, worin X eine 1 bis 10 Kohlenstoffatome umfassende Alkylengruppe darstellt und Y eine 1 bis 3 Kohlenstoffatome umfassende Alkylengruppe darstellt, während R₂ ein Wasserstoffatom darstellt.

4. Sensor gemäß Anspruch 3, bei dem die sich wiederholende Siloxaneinheit der Formel (I) entspricht, worin X eine Propylengruppe darstellt und Y eine Methylengruppe darstellt, während R₂ ein Wasserstoffatom darstellt.

5. Sensor gemäß einem der vorangehenden Ansprüche, bei dem die sich wiederholende Siloxaneinheit einer der nachstehenden Formeln (Ia), (Ib) und (Ic) entspricht:

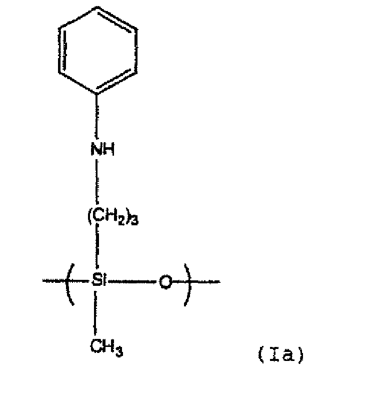

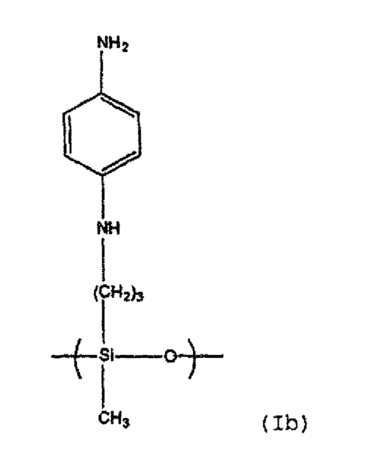

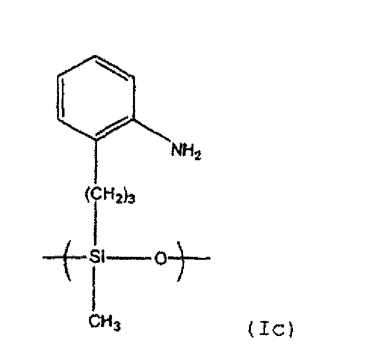

6. Sensor gemäß einem der vorangehenden Ansprüche, bei dem das Polymer ein Homopolymer ist.

7. Sensor gemäß einem der vorangehenden Ansprüche 1 bis 5, bei dem das Polymer ein Copolymer ist, das zwei verschiedene, sich wiederholende Siloxaneinheiten umfasst, wobei eine erste Einheit der Formel (I) entspricht, worin X, Y, R₁ und R₂ dieselbe Bedeutung wie voranstehend aufweisen, und eine zweite Einheit der nachstehenden Formel (III) entspricht: 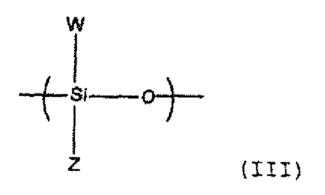 worin W und Z, die gleich oder verschieden sein können, ein Wasserstoffatom oder eine 1 bis 30 Kohlenstoffatome umfassende, gesättigte oder ungesättigte, gerade, verzweigte oder cyclische Kohlenwasserstoffgruppe darstellen.

8. Sensor gemäß Anspruch 7, bei dem die zweite Siloxaneinheit eine Dihydrogenosiloxaneinheit, eine Methylhydrogenosiloxaneinheit oder eine Dimethylsiloxaneinheit ist.

9. Sensor gemäß Anspruch 6, bei dem das Polymer ein Homopolymer ist, das durch die sich wiederholende Siloxaneinheit der nachstehenden Formel (Ia) dargestellt wird:

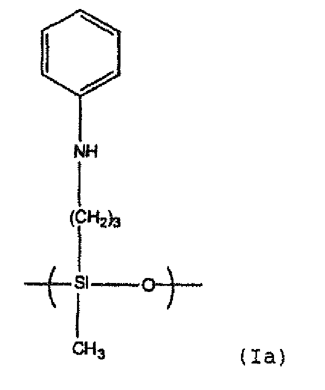

10. Sensor gemäß Anspruch 8, bei dem das Polymer ein Copolymer ist, das eine erste, der nachstehenden Formel (Ia) entsprechende, sich wiederholende Siloxaneinheit 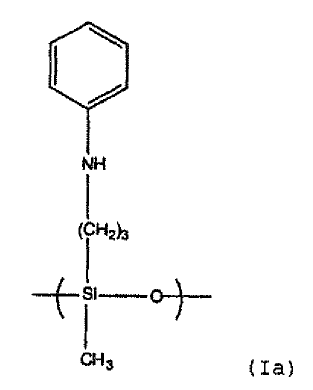 und eine zweite Dimethylsiloxaneinheit umfasst.

11. Sensor gemäß Anspruch 8, bei dem das Polymer ein Copolymer ist, das eine erste, der nachstehenden Formel (Ib) entsprechende, sich wiederholende Siloxaneinheit 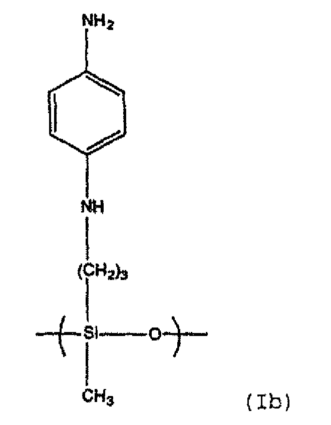 und eine zweite Dimethylsiloxaneinheit umfasst.

12. Sensor gemäß Anspruch 8, bei dem das Polymer ein Copolymer ist, das eine erste, der nachstehenden Formel (Ic) entsprechende, sich wiederholende Siloxaneinheit 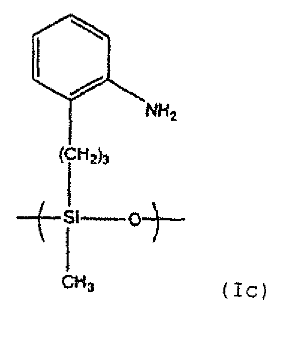 und eine zweite Dimethylsiloxaneinheit umfasst.

13. Sensor gemäß einem der Ansprüche 10 bis 12, bei dem das Copolymer ein zufallsverteiltes, alternierendes oder ein Blockcopolymer ist.

14. Sensor gemäß einem der Ansprüche 10 bis 11, bei dem die erste sich wiederholende Einheit zahlenmäßig ungefähr 47 % der das Copolymer bildenden, sich wiederholenden Einheiten darstellt, während die zweite sich wiederholende Einheit zahlenmäßig ungefähr 53 % der das Copolymer bildenden Einheiten darstellt.

15. Sensor gemäß einem der vorangehenden Ansprüche, bei dem das Polymer oder der Verbund in Form eines eine oder beide Flächen eines Substrats bedeckenden, dünnen Filmes vorliegt.

16. Sensor gemäß Anspruch 15, bei dem der dünne Film in der Dicke 10 Ångström bis 100 Mikron misst.

17. Sensor gemäß einem der vorangehenden Ansprüche, wobei der Sensor ein gravimetrischer Sensor ist.

18. Sensor gemäß einem der vorangehenden Ansprüche, wobei der Sensor ein Quarzmikrowaagensensor ist.

19. Mehrfachsensor, der einen oder mehrere einzelne, miteinander zusammengebaute Sensoren umfasst und bei dem wenigstens einer dieser einzelnen Sensoren ein chemischer Sensor gemäß einem der Ansprüche 1 bis 18 ist.

20. Verwendung eines chemischen Sensors gemäß einem der Ansprüche 1 bis 18 oder eines Mehrfachsensors gemäß Anspruch 19 zum Nachweis oder zur Analyse einer oder mehrerer Nitroverbindungen.

21. Verwendung gemäß Anspruch 20, wobei die nitrierte(n) Verbindung(en) aus nitroaromatischen Verbindungen, Nitraminen, Nitrosaminen und Salpetersäureestern ausgewählt sind.

22. Verwendung gemäß Anspruch 21, wobei die nachzuweisende(n) nitrierte(n) Verbindung(en) gasförmig sind.

23. Verwendung gemäß einem der Ansprüche 20 bis 22, wobei die nitrierte(n) Verbindung(en) aus Nitrobenzol, Dinitrobenzol, Trinitrobenzol, Nitrotoluol, Dinitrotoluol, Trinitrotoluol, Dinitrofluorbenzol, Dinitrotrifluormethoxybenzol, Aminodinitrotoluol, Dinitrotrifluormethylbenzol, Chlordinitrotrifluormethylbenzol, Hexanitrostilben, Trinitrophenol, Cyclotetramethylentetranitramin, Cyclotrimethylentrinitramin, Trinitrophenylmethylnitramin, Nitrosodimethylamin, Pentrit, Ethylenglykoldinitrat, Diethylenglykoldinitrat, Nitroglycerin oder Nitroguanidin ausgewählt sind.

24. Verwendung gemäß einem der Ansprüche 20 bis 23 zum Nachweis von Explosivstoffen.
